(19) Europäisches Patentamt
European Patent Office
Office européen des brevets

(11) Veröffentlichungsnummer: **0 134 986**
**B1**

(12) # EUROPÄISCHE PATENTSCHRIFT

(45) Veröffentlichungstag der Patentschrift: **27.12.89**

(51) Int. Cl.⁴: **C 07 K 7/06,** A 61 K 37/02, A 23 K 1/16

(21) Anmeldenummer: **84108009.6**

(22) Anmeldetag: **09.07.84**

(54) **Biologisch aktive Heptapeptide, Mittel, die diese enthalten und Anwendungsverfahren.**

(30) Priorität: **13.07.83 GB 8319174**

(43) Veröffentlichungstag der Anmeldung: **27.03.85 Patentblatt 85/13**

(45) Bekanntmachung des Hinweises auf die Patenterteilung: **27.12.89 Patentblatt 89/52**

(84) Benannte Vertragsstaaten: **AT BE CH DE FR GB IT LI NL SE**

(56) Entgegenhaltungen:
**GB-A-2 130 590**

**CHEMICAL ABSTRACTS, Band 103, Nr. 15, 14. Oktober 1985, Seite 769, Zusammenfassung Nr. 123889z, Columbus, Ohio, US; R. DE CASTIGLIONE et al.: "Structure synthesis and preliminary biological results of tryptophyllin-7 and analogs"**
**CHEMICAL ABSTRACTS, Band 101, Nr. 11, 10. September 1984, Seite 232, Zusammenfassung Nr. 85905a, Columbus, Ohio, US; C. PIER et al.: "Primary structure of tryptophan-containing peptides from skin extracts of phyllomedusa rhodei (tryptophyllins)"**
**CHEMICAL ABSTRACTS, Band 103, Nr. 7, 19. August 1985, Seite 616, Zusammenfassung Nr. 54439p, Columbus, Ohio, US; G. PERSEO et al.: "Synthesis of tryptophyllin-7 and analogs"**

(73) Patentinhaber: **FARMITALIA CARLO ERBA S.r.L., Via Carlo Imbonati 24, I-20159 Milano (IT)**

(72) Erfinder: **de Castiglione, Roberto, Via Domenichino, 38, I-20149 Mailand (IT)**
Erfinder: **Perseo, Giuseppe, Via Monte Bianco, 66, I-20033 Desion (IT)**
Erfinder: **Gigli, Mauro, Via Vezza d'Oglio, 10, I-20139 Mailand (IT)**
Erfinder: **Hecht, Barbara, Via Barzini, 14, I-20125 Mailand (IT)**

(74) Vertreter: **Eitle, Werner, Dipl.- Ing., Hoffmann, Eitle & Partner Patentanwälte Arabellastrasse 4, D-8000 München 81 (DE)**

EP 0 134 986 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft biologisch aktive Heptapeptide, Mittel, die diese enthalten und Anwendungsverfahren. Insbesondere betrifft die Erfindung biologisch aktive Peptide und deren pharmazeutisch oder tierarzneilich annehmbaren Salze, Verfahren zu deren Herstellung sowie Anwendungsverfahren und pharmazeutisch oder tierarzneiliche Mittel, die diese Heptapeptide enthalten.

In der nachfolgenden Beschreibung entsprechen die Symbole und Abkürzungen den üblicherweise in der Peptidchemie verwendeten; hierzu wird auf Biochemistry (1975) *14*, 449 verwiesen.

Die Erfindung betrifft Peptide der allgemeinen Formel:

X-Val-Pro-Pro-Leu-Gly-Trp-A-Y,

worin bedeuten:

X ein Wasserstoffatom oder eine Schutzgruppe für den endständigen Stickstoff vom Acyl-, aliphatischen Urethan-, aromatischen Urethan, Alkyl- oder Aralkyltyp;

A ein neutraler L-α-Aminosäurerest; und

Y eine Hydroxygruppe, eine Aminogruppe oder eine Gruppe der Formel OR, NHR, $NR_2$ oder NH-NH-R', worin

R eine geradkettige, verzweigte oder cyclische (einschließlich kondensierte Ring- oder Brückenring-) Alkylgruppe mit bis zu 11 Kohlenstoffatomen, welche nichtsubstituiert oder mit einer Hydroxy- oder Aminogruppe oder einem Halogenatom substituiert ist, eine Aralkylgruppe mit 7 bis 14 Kohlenstoffatomen oder eine Phenylgruppe bedeutet; und

R' ein Wasserstoffatom, eine beliebige Gruppe mit der Bedeutung von R, eine geradkettige, verzweigte oder cyclische aliphatische Acylgruppe mit 1 bis 11 Kohlenstoffatomen, welche nichtsubstituiert oder durch eine Hydroxy- oder eine Aminogruppe oder ein Halogenatom substituiert ist, eine aromatische Acylgruppe, welche nichtsubstituiert oder mit einer Hydroxy- oder Aminogruppe oder einem Halogenatom substituiert ist, eine geradkettige, verzweigte oder cyclische Gruppe vom Urethantyp mit 3 bis 11 Kohlenstoffatomen, oder eine aromatische Urethangruppe bedeutet.

Bevorzugte terminale N-Schutzgruppen, welche X darstellen kann, umfassen (vom Acyltyp) Formyl-, Acetyl-, Trifluoracetyl-, Propionyl- und Benzoylgruppen; (vom aromatischen Urethantyp) Benzyloxycarbonyl- (Z), 4-Nitrobenzyloxycarbonyl-, 4-Methoxybenzyloxycarbonyl-, 2,4-Dichlorbenzyloxycarbonyl-, 2-Brombenzyloxycarbonyl-, 9-Fluorenylmethoxycarbonyl- (Fmoc) und 3,5-Dimethoxy-α,α'-dimethylbenzyloxycarbonyl- (Ddz) Gruppen; (vom aliphatischen Urethantyp) t-Butoxycarbonyl-, 1-Methylcyclobutoxycarbonyl, Adamantyloxycarbonyl-, Isobornyloxycarbonyl- und Methylsulfonylethoxycarbonyl- (Msc) Gruppen; und (vom Alkyl- und Aralkyltyp) Trityl-, Benzyl-, Methyl- und Isopropylgruppen.

Bevorzugte L-α-Aminosäurereste, welche A darstellen kann, umfassen Met, Nle, Ile, Leu und Phe.

Bevorzugte Gruppen, die R darstellen kann, umfassen Methyl, Ethyl, n-Propyl, Isopropyl, n-Butyl, s-Butyl, Isobutyl, t-Butyl, 2,2,2-Trifluorethyl, Cyclohexyl, Adamantyl, Phenyl, Benzyl, Phenethyl und Fluorenyl.

Beispiele für Acylgruppen, die R' darstellen kann, umfassen Formyl-, Acetyl-, Trifluoracetyl-, Propionyl-, Butyryl-, Adamantylcarbonyl-, Benzoyl-, Phenylacetyl- und Cinnamylgruppen. Die aliphatischen und aromatischen Gruppen vom Urethantyp, die R' darstellen kann, sind vorzugsweise Gruppen, die als bevorzugte terminale N-Schutzgruppen X vom aliphatischen und aromatischen Urethantyp genannt worden sind.

In den Substituenten der Alkyl- und Acylgruppen, die Y darstellen kann, umfaßt die Bezeichnung "Halogen" Chlor-, Brom- und Fluoratome sowie Jodatom.

Die Erfindung umfaßt auch Salze der erfindungsgemäßen Peptide mit pharmazeutisch annehmbaren Säuren oder Basen. Diese Säureadditionssalze können von einer Vielzahl anorganischer und organischer Säuren abgeleitet sein, wie z. B. Schwefel-, Phosphor-, Salz-, Bromwasserstoff-, Jodwasserstoff-, Salpeter-, Sulfamin-, Zitronen-, Milch-, Brenztrauben-, Oxal-, Malein-, Bernstein-, Wein-, Zimt-, Essig-, Trifluoressig-, Benzol-, Salicyl-, Glucon- und Ascorbinsäure. Additionssalze mit einer Base können von einer Vielzahl anorganischer oder organischer Basen abgeleitet sein, wie z. B. Natriumhydroxid, Kaliumhydroxid, Diethylamin, Triethylamin und Dicyclohexylamin.

Die Synthese der erfindungsgemäßen Peptide erfolgt nach klassischen Methoden. Die Synthese besteht im wesentlichen aus geeigneten sukzessiven Kondensationen von geschützten Aminosäuren oder Peptiden. Die Kondensationen werden so ausgeführt, daß die erhaltenen Peptide die gewünschte Sequenz von sieben Aminosäureresten aufweisen. Bei den Aminosäuren und Peptiden, die nach Methoden, die in der Polypeptidchemie bekannt sind, kondensiert werden, weisen die Amino- und Carboxylgruppen, die nicht an der Bildung von Peptidbindungen beteiligt sind, geeignete Schutzgruppen auf. Die Schutzgruppen können durch Ansäuerung (Acidolyse), Verseifung und Hydrogenolyse entfernt werden. Die folgenden Gruppen eignen sich zum Schutz von Aminogruppen: Benzyloxycarbonyl, t-Butoxycarbonyl, Trityl, Formyl, Trifluoroacetyl, o-Nitrophenylsulfenyl, 4-Methoxybenzyloxycarbonyl, 9-Fluorenylmethoxycarbonyl, 3,5-Dimethoxy-α,α'-dimethylbenzyloxycarbonyl und Methylsulfonylethoxycarbonyl. Die folgenden Gruppen werden zum Schutz der Carboxygruppe verwendet: Methyl, Ethyl, t-Butyl, Benzyl, p-Nitrobenzyl und Fluorenylmethyl.

Die Kondensation zwischen einer Aminogruppe eines Moleküls und einer Carboxylgruppe eines anderen Moleküls unter Bildung einer Peptidbindung kann über ein aktiviertes Acyl-Derivat erfolgen, wie z. B. einem gemischten Anhydrid, einem Azid oder einem aktivierten Ester, oder durch direkte Kondensation zwischen einer

freien Aminogruppe und einer freien Carboxylgruppe, in Gegenwart eines Kondensationsmittels, wie Dicyclohexylcarbodiimid, allein oder zusammen mit einem Agens, welches die Racemisierung verhindert, wie z. B. N-Hydroxysuccinimid oder 1-Hydroxybenzotriazol. Die Kondensation kann in einem Lösungsmittel ausgeführt werden, wie z. B. Dimethylformamid, Pyridin, Acetonitril, Tetrahydrofuran oder N-Methyl-2-pyrrolidon. Die Reaktionstemperatur kann zwischen -30°C bis zu Raumtemperatur betragen. Die Reaktionszeit liegt im allgemeinen zwischen 1 und 120 h. Das Syntheseschema, die Schutzgruppen und die Kondensationsmittel sind so ausgewählt, daß die Gefahr einer Racemisierung vermieden wird.

Reaktionen zur Entfernung der Schutzgruppen werden nach Methoden, wie sie inder Polypeptidchemie bekannt sind, durchgeführt. Peptide, in denen Y die Gruppe OR bedeutet, werden z. B. hergestellt, indem man von einer C-terminalen Aminosäure ausgeht, die mit einem geeigneten Alkohol verestert ist. Peptide, in denen Y die Gruppe OH darstellt, können z. B. hergestellt werden durch Hydrolyse von Peptiden, in welchen Y die Gruppe OR darstellt. Peptide, in denen Y die Gruppen $NH_2$, NHR oder $NR_2$ darstellt, können durch Ammonolyse der entsprechenden Ester hergestellt werden, oder indem man von einer C-terminalen Aminosäure ausgeht, die mit einem geeigneten Amin amidiert ist. Hydrazido- oder substituierte Hydrazidoderivate gemäß der Erfindung werden hergestellt durch Kondensation des N-geschützten Peptids oder der entsprechenden Aminosäure mit einem entsprechend substituierten Hydrazin, wie Benzylcarbazat, t-Butylcarbazat, Adamantylcarbazat, Phenylhydrazin oder Adamantylhydrazin oder indem man das N-geschützte Peptid- oder entsprechende Aminosäurehydrazid mit einem geeigneten Alkylierungsmittel, wie z. B. einem Alkylchlorid, oder mit einem geeigneten Acylierungsmittel, wie Benzylchlorformiat, t-Butylchloroformiat, Di-t-Butyldicarbonat oder Adamantylfluorformiat umsetzt.

Die endgültige Kondensation in der Herstellung eines Peptids gemäß der Erfindung erfolgt vorzugsweise zwischen einer Verbindung der Formel II:

X-Val-Pro-Pro-OH,

in Gegenwart eines Kondensationsmittels, wie Dicyclohexylcarbodiimid, allein oder zusammen mit einem Mittel, welches die Racemisierung verhindert, oder einem gemischten Anhydrid, aktivierten Ester oder Azidderivat der Verbindung der Formel II, wie sie oben angegeben ist, und einer Verbindung der Formel III:

H-Leu-Gly-Trp-A-Y,

worin A, X und Y die vorstehend angegebene Bedeutung haben, mit Ausnahme, daß X nicht ein Wasserstoffatom und Y nicht eine Hydrazingruppe darstellt.

Verbindungen gemäß der Erfindung zeigen eine überraschende wachstumsfördernde Aktivität bei Tieren, wie dies sowohl durch in vivo- als auch in vitro-Testsysteme der Proteinsynthese des Lebergewebes festgestellt worden ist (beschrieben von K. Kämmerer und A. Dey-Hazra, Vet. Med. Nachr. Nr. 2 (1980), Seiten 99 bis 112). Im weiteren konnte dies auch durch den dosisabhängigen Anstieg der Gewichtszunahme und Futtereffizienz nach subkutaner oder oraler Verabreichung bestätigt werden.

**Proteinsynthese bei in vivo- und in vitro-Tests**

Der Aufzuchtversuch wurde über eine Zeitdauer von 4 Wochen durchgeführt, wobei Gruppen von 6 männlichen Ratten (Wistar, Hagemann, Extertal) aufgeteilt in Untergruppen von 3 Tieren, in Makrolon-Käfigen mit Holzspänen als Streu, eingesetzt wurden.

Wasser und Futter (Altromin-Diät in Mehlform welche 19 % Rohprotein, enthält): ad libitum.

Die Peptide gemäß der Erfindung wurden in Lösung (ausgehend von einer Stammlösung von 100 ng/ml) s.c., täglich in Dosen von 10, 50 und 100 ng/kg, unter Verwendung von physiologischer Kochsalzlösung (normal) als Verdünnungsmittel verabreicht.

**Herstellung von Gewebsproben**

Es wurden 3 g Leber in 9 ml TKM-Puffer/Saccharose-Lösung, auf Eis gekühlt, in einem Potter-Homogenisator bei 600 Upm, 2 min lang homogenisiert; daraufhin wurde das Homogenisat bei 4°C in einer Ultrazentrifuge mit 10.000 g, 20 min lang, zentrifugiert, der Überstand abgenommen, welcher den mikrosomalen Zellsaft enthält. (Anmerkung: Der vorstehende TKM-Puffer stellt einen Puffer aus Tris-Cl, KCl und $MgC_2$ dar.)

**Arbeitsverfahren**

Nach Berechnung des Proteingehaltes im mikrosomalen Zellsaft mittels der Biuret-Methode wurde die Proteinkonzentration mit TKM-Puffer (siehe oben) auf 1 mg/ml eingestellt. Daraufhin folgte eine weitere Verdünnung mit bidestilliertem Wasser auf 0,25 mg Protein pro ml des mikrosomalen Zellsaftes.

Daraufhin wurden Anteile von 0,15 ml Reaktionsmedium und 0,05 ml (50 mcg) Pyruvatkinase-Lösung, sowie

0,1 ml $^{14}C$-Aminosäure gemisch (= 1 µCi) zugegeben. Das Inkubationsvolumen betrug jeweils 1 ml.

Nach 35 minütiger Inkubation bei 37C in einem Wasserbad wurde eine Proteinfällung durch Zugabe von 2 ml Trichloressigsäure (10-%-ig) durchgeführt. Der Niederschlag wurde durch mehrmalige Zugabe von Trichloressigsäure und anschließende Zentrifugation (3.600 g/5 min) gewaschen, bis der Überstand keine Radioaktivität mehr enthielt.

Der Rückstand wurde in 1,0 ml Lumasolve ® aufgelöst und über Nacht bei 37°C stehen gelassen, bis die Lösung klar ist. (Lumasolve stellt ein organisches quaternäres Ammoniumhydroxid dar.)

Die Bestimmung der Präparation erfolgte in einem PRIAS-Flüssig-Szintillationszähler PL (1,0 ml + 5 ml Szintillationsflüssigkeit).

Das Verfahren wird im einzelnen in der vorstehend genannten Publikation von Kämmerer et al. beschrieben.

**Tabelle 1**

Ergebnisse der Bestimmungen zur Proteinsynthesegeschwindigkeit im Lebergewebe von männlichen Ratten nach 4-wöchiger Behandlung (tägliche s.c.-Injektion)

| Behandlung mit | Dosis (ng/kg) | CMP | Δ% Kontrollgruppe von Ratten unbehandelt | behandelt |
|---|---|---|---|---|
| Verbindung von Beispiel 1 (TK7) | 10 | 14072 | +17,7 | +36,1 |
| | 50 | 15093 | +26,2 | +46,0 |
| | 100 | 16180 | +35,3 | +56,5 |
| Verbindung von Beispiel 2 (TK7D) | 10 | 13377 | +11,9 | +29,4 |
| | 50 | 14280 | +19,4 | +38,2 |
| | 100 | 16866 | +41,0 | +63,2 |
| Unbehandelte Kontrollgruppe | - | 11958 | - | - |
| Behandelte Kontrollgruppe | - | 10336 | -13,6 | - |

CPM = Counts pro Minute

**Kontrollgruppe**

Die Ratten, denen s.c. physiologische Natriumchloridlösung verabreicht worden war, zeigten im Vergleich zur unbehandelten Kontrollgruppe eine verminderte Aktivität der Proteinsynthese von 13,6 %. Dieser Unterschied wurde statistisch bestätigt.

Die berechneten Proteinsyntheseraten unter Anwendung der in den Beispielen 1 und 2 hergestellten Verbindungen unterscheiden sich signifikant von den zwei Kontrollgruppen.

Im weiteren wurde festgestellt, daß die Zunahme der Proteinsyntheseraten im Verhältnis zu der jeweiligen Dosis erfolgt.

Die erfindungsgemäßen Verbindungen besitzen auch interessante endocrinologische Aktivitäten, wie z. B. eine Freisetzung von Prolactin und luteinisierendem Hormon. Im weiteren weisen sie eine Aktivität auf das zentrale Nervensystem auf, insbesondere als Sedativ-Hypnotika. Sie können bei Ratten die Spontanaktivität reduzieren und das Schlafverhalten beeinflussen.

Die Erfindung umfaßt auch die Schaffung eines Verfahrens zur Wachstumsförderung bei Tieren und zur Verbesserung ihrer Futterverwertung durch Verabreichung der erfindungsgemäßen Verbindungen an dieselben.

Dieses Verfahren ist besonders geeignet für landwirtschaftliche Nutztiere, wie Geflügel, Wiederkäuer, Schweine und Kaninchen. Obwohl bei sämtlichen Geflügelarten, nämlich Hühnern, Truthähnen, Gänsen, Enten, Perlhühnern (Guineas), Fasanen und Wachteln, eine erhöhte Wachstumsrate und eine verbesserte Futterverwertung festzustellen sein dürfte, ist das erfindungsgemäße Verfahren besonders geeignet für Masthähnchen und Mastputen.

Von den Wiederkäuern, nämlich Rinder, Schafe und Ziegen, eignet sich das Verfahren insbesondere für Rinder, bevorzugt für Stiere.

Nach dem erfindungsgemäßen Verfahren wird eine Verbindung gemäß der Erfindung in die Futterrationen, die für die Tiere bestimmt sind, in einer Konzentration von ca. 2 - 40 meg/Tonne Futter, vorzugsweise ca. 4 - 20 meg/Tonne eingemischt. Während der gesamten Wachstumsperiode läßt man die Tiere nach Belieben fressen.

Es gibt zahlreiche spezialisierte Futterrationen für die verschiedenen Tierarten. Die erfindungsgemäßen Verbindungen können bei sämtlichen der bekannten Rationen eingesetzt werden.

Die Bezeichnung "Futterrationen" bedeutet das den Tieren zur Verfügung gestellte Futter; die Erfindung soll dadurch in keiner Weise begrenzt werden. Vorzugsweise wird die erfindungsgemäße Verbindung gründlich in die

Futterration eingemischt. so daß sie in derselben einheitlich verteilt ist. In der praktischen Anwendung der Erfindung können beliebige bekannte Futterrationen eingesetzt werden; die Erfindung soll nicht durch die Formulierung der Rationen begrenzt werden.

Die Futterrationen sind so formuliert, daß den Tieren die essentiellen Nährstoffe, Minerale, Vitamine, Trockensubstanz etc. zur Verfügung gestellt werden.

Die Formulierungen dieser Rationen können in üblicher Weise von den Fachleuten für Ernährungskunde ausgearbeitet werden.

Ein weiteres Verfahren zur Verabreichung der erfindungsgemäßen Verbindungen besteht in der subkutanen Implantierung, z. B. in Form von Pellets, mit einer täglichen Freisetzungsrate der Wirkstoffe in einem Dosierungsbereich von 1 bis 100 ng/kg, vorzugsweise 10 bis 50 ng/kg, injiziert wird.

Somit soll das Verfahren zur Verabreichung der erfindungsgemäßen Verbindungen nicht auf eine spezielle Verabreichungsmethode beschränkt sein.

Im weiteren umfaßt die Erfindung pharmazeutische oder tiermedizinische Zubereitungen, welche eine Verbindung gemäß der Erfindung oder ein pharmazeutisch oder tierarzneilich annehmbares Salz derselben im Gemisch mit einem pharmazeutisch oder tierarzneilich annehmbaren Verdünnungsmittel oder Träger enthalten; außerdem können diese Präparationen direkt oder in verzögerter Weise den Wirkstoff freisetzen.

Tierarzneilich annehmbare Träger betreffen ein eßbares Material, zu dem die erfindungsgemäßen Peptide zugegeben werden, um eine einheitliche Inkorporierung dieser Peptide im Futter zu erleichtern.

Die aktiven Peptide werden von dem eßbaren Material entweder adsorbiert, oder dasselbe damit imprägniert oder beschichtet, und zwar in einer Weise, daß das genannte Material die aktiven Peptide verteilt und physikalisch trägt.

Bevorzugte Peptide gemäß der Erfindung sind folgende:

H-Val-Pro-Pro-Leu-Gly-Trp-Met-OH
H-Val-Pro-Pro-Leu-Gly-Trp-Met-OMe
H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-OMe
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

Die folgenden Beispiele sollen die Erfindung näher erläutern. Die $R_f$-Werte wurden auf vorbeschichteten Silicagelplatten 60 $F_{254}$ (Merck), Schichtdicke 0,25 mm, Länge 20 cm, unter Verwendung der folgenden Entwicklungssysteme bestimmt:

System A: Benzol/Benzin (60 - 80)/Ethylacetat = 70/10/40 (nach dem Volumen).
System B: Benzol/Ethylacetat/Essigsäure/Wasser = 100/100/20/10 nach dem Volumen (obere Phase).
System C: Benzol/Ethylacetat/Essigsäure/Wasser = 100/100/40/15 nach dem Volumen (obere Phase).
System D: n-Butanol/Essigsäure/Wasser = 4/1/1 nach dem Volumen.

"E.Merck" stellt einen Markennamen dar.

TLC-Analysen wurden bei Temperaturen im Bereich von 18 bis 25°C durchgeführt: die $R_f$-Werte können somit um ± 5 % variieren. Die Schmelzpunkte wurden in offenen Kapillaren mit einem Tottoli-Apparat bestimmt und sind nicht korrigiert. Die meisten der Derivate erweichen und zersetzen sich vor dem Schmelzen. Die Lösungsmittel zur Kristallisation, Ausfällung oder zum Homogenisieren werden in Klammern angegeben.

Eine Hochspannungs-Papier-Elektrophorese wurde mit einem Pherograph-Original-Frankfurt Typ 64-Gerät auf Schleicher und Schüll-Papier N. 2317 bei einem pH von 1,2 (Ameisensäure: Essigsäure: Wasser = 123 : 100 : 777 nach dem Volumen) bei 1600 V (40 V/cm), und bei einem pH-Wert von 5,8 (Pyridin: Essigsäure: Wasser = 450 : 50 : 4500 nach dem Volumen) bei 1400 V (32,5 V/cm) durchgeführt. Die Produkte wurden nach ihrer Mobilität in bezug zu Glu bei einem pH-Wert von 1,2 ($E_{1,2}$), und bei einem pH-Wert von 5,8 ($E_{5,8}$) charakterisiert. Die Symbole und Abkürzungen, die in den folgenden Beispielen verwendet werden, bedeuten:

AcOET, Ethylacetat; BOC, t-Butoxycarbonyl; Bzl, Benzyl; d, Zersetzung; DMF, Dimethylformamid; $Et_20$, Diethylether; HC1/THF, Chlorwasserstoff in Tetrahydrofuran; $iPr_2O$, Diisopropylether; iPrOH, Isopropanol; Me, Methyl; MeOH, Methanol; NMM, N-Methylmorpholin; PE, Petroleumether; THF, Tetrahydrofuran; TLC, Dünnschichtchromatographie.

**Beispiel 1**

**Herstellung von H-Val-Pro-Pro-Leu-Gly-Trp-Met-OH Ã HC1 TK7 (XIII)**

**Schritt 1: BOC-Trp-Met-OMe (I)**

Zu einer Lösung von 30,43 g (100 mMol) BOC-Trp-OH in 200 ml wasserfreiem THF werden nacheinander 11,2 ml (100 mMol) NMM und 9,9 ml Ethylchloroformiat bei einer Temperatur von -12°C zugegeben. Nach 2-minütigem Rühren bei dieser Temperatur wird eine kalte Lösung von 19,96 g (100 mMol) HCl · H-Met-OMe (C.A. Dekker et al., T. Biol. Chem. 180, 155 (1949) und 11,2 ml NMM (100 mMol) in 150 ml DMF zugegeben. Das Reaktionsgemisch wird 45 min lang bei -12°C und 90 min lang bei 0 bis 15°C gerührt, dann werden die Salze abfiltriert und das Filtrat im Vakuum eingedampft. Der Rückstand wird in Ethylacetat aufgelöst und sukzessive mehrere Male mit gesättigten Natriumchloridlösungen von 1M Zitronensäure, 1M Natriumbicarbonat und Wasser gewaschen. Die organische Schicht wird dann über wasserfreiem Natriumsulfat getrocknet und das Lösungsmittel mit Vakuum entfernt.

39,12g (87 % Ausbeute) der Verbindung I wurden aus iPrOH/$iPr_2O$/PE erhalten; Schmelzpunkt 95 bis 97°C; $[\alpha]_D^{20}$ = -25,1° (c = 1, MeOH); $R_{fA}$ 0,73, $R_{fB}$ 0,76.

**Schritt 2: HCl · H-Trp-Met-OMe (II)**

39,00 g (86,75 mMol) BOC-Trp-Met-OMe (I) wurden in 390 ml Ameisensäure bei Raumtemperatur aufgelöst. Nach vollständiger BOC-Entfernung (verfolgt mit TLC) wurde das Lösungsmittel im Vakuum bei 30°C eingedampft. Der Rückstand wurde in Methanol, das auf 0°C abgekühlt war, aufgelöst und 34,7 ml (104,1 mMol) einer 3M HCl/THF-Lösung zugegeben. Die Lösungsmittel wurden im Vakuum entfernt; 33,48 g der Verbindung II wurden in quantitativer Ausbeute als Öl erhalten, $R_{fD}$ 0,71, $E_{1.2}$ 0,82.

**Schritt 3: BOC-Gly-Trp-Met-OMe (III)**

Ausgehend von 15,20 g (86,75 mMol) BOC-Gly-OH und 33,48 g (86, 75 mMol) HC1 · H-Trp-Met-OMe (II), wobei wie in Schritt 1 verfahren wurde, jedoch anstelle von Ethylacetat während der Isolierung des Produktes Chloroform verwendet wurde, wurden 26,37 g (60-%-ige Ausbeute) der Verbindung III aus ACOEt/$Et_2O$/$iPr_2O$ erhalten: Schmelzpunkt 140 bis 145°C; $[\alpha]^{20}_D$ = -30 (c = 1, MeOH); $R_{fA}$ 0,27; $R_{fB}$ 0,56; $R_{fC}$ 0,77.

**Schritt 4: HCl · H-Gly-Trp-Met-OMe (IV)**

Ausgehend von 26,20 g (51,71 mMol) BOC-Gly-Trp-Met-OMe (III), wobei wie im Schritt 2 beschrieben verfahren wurde, wurden 21,76 g (95-%-ige Ausbeute) der Verbindung IV aus iPrOH/$iPr_2O$ erhalten: Schmelzpunkt 195°C (Zersetzung); [α]20D = -12,9° (c = 1, MeOH); RfD 0,49, E1.2 0,8 h.

**Schritt 5: BOC-Leu-Gry-Met-OMe (V)**

Zu einer Lösung von 12,16 g (48,76 mMol) BOC-Leu-OH in 120 ml wasserfreiem THF wurden nacheinander 5,5 ml (48,76 mMol) NMM und 4,8 ml (48,76 mMol) Ethylchloroformiat bei einer Temperatur von -12°C zugegeben. Nach 2-minütigem Rühren bei dieser Temperatur wurde eine kalte Lösung von 21,6 g (48,76 mMol) HCl · H-Gly-Trp-Met-OMe (IV) und 5,5 ml (48,76 mMol) NMM in 100 ml DMF zugegeben. Das Reaktionsgemisch wurde 1 h lang bei -12°C und 2 h lang bei 0 bis 15°C gerührt, dann wurden die Salze abfiltriert und das Filtrat im Vakuum eingedampft. Das Rohprodukt wurde durch Säulenchromatographie über Silicagel (Merck), 0,040 bis 0,063 mm, Eluierung mit AcOEt, gereinigt. Aus AcOEt/$Et_2O$/PE wurden 18,13 g (60-%-ige Ausbeute) der Verbindung V erhalten: Schmelzpunkt 110°C $[\alpha]^{20}_D$ = -31,6° (c = 1, MeOH); $R_{fA}$ 0,14; $R_{fB}$ 0,54.

**Schritt 6: HCl · H-Leu-Gly-Trp-Met-OMe (VI)**

18 g (29,04 mMol) BOC-Leu-Gly-Trp-Met-OMe (V) wurden in 290 ml einer gesättigten Lösung von Chlorwasserstoff in Eisessig, zu welcher 18 ml Anisol und 9 ml 2-Mercaptoethanol zugegeben worden waren, zugegeben. Nach 30 Minuten bei Raumtemperatur war die BOC-Entfernung vollständig; das Lösungsmittel wurde bei 30°C im Vakuum entfernt. Das Rohprodukt wurde durch Säulenchromatographie auf Silicagel (Merck) 0,040 bis 0,063 mm, Eluierung mit Chloroform: Methanol = 8 : 2 gereinigt. Aus iPrOH/$iPr_2O$ wurden 12,44 g (77-%-ige Ausbeute) der Verbindung VI erhalten: Schmelzpunkt 170°C; $[\alpha]^{20}_D$ = -9,9° (c = 1, MeOH); $R_{fD}$ 0,62; $E_{1,2}$ 0,71.

**Schritt 7: BOC-Pro-Pro-OBzl (VII)**

Zu einer Lösung aus 21,52 g (100 mMol) BOC-ProOH in 200 ml wasserfreiem THF wurden nacheinander 11,2 ml NMM und 13,3 ml Iso-butylchloroformiat bei einer Temperatur von -10°C zugegeben. Nach 3-minütigem Rühren bei dieser Temperatur wurde eine kalte Lösung aus 24,17 g (100 mMol) HCl · H-ProOBzl (J. Ramachandran und C.H. Li, J. Org. Chem. (1963), *28*, 1973) und 11,2 ml (100 mMol) NMM in 150 ml DMF zugegeben. Das Reaktionsgemisch wurde 1 h lang bei -10°C und 2 h lang bei 0 bis 15°C gerührt, die Salze wurden abfiltriert, das Filtrat im Vakuum eingedampft. Der Rückstand wurde in Ethylacetat aufgelöst und nacheinander mehrere Male mit gesättigten Natriumchloridlösungen von 1M Zitronensäure, 1M Natriumbicarbonat und Wasser gewaschen. Die organische Schicht wurde über wasserfreiem Natriumsulfat getrocknet und das Lösungsmittel im Vakuum entfernt; es wurden 34,21 g (85-%-ige Ausbeute) der Verbindung VII als Öl erhalten. $R_{fA}$ = 0,62.

**Schritt 8: HCl · H-Pro-Pro-OBzl (VIII)**

34,21 g (85 mMol) BOC-Pro-Pro-OBzl (VII) wurden in 342 ml einer gesättigten Lösung von Chlorwasserstoff in Essigsäure bei Raumtemperatur aufgelöst. Nach 30 Minuten war die BOC-Entfernung vollständig; das Lösungsmittel wurde im Vakuum entfernt. Aus iPrOH/AcOEt wurden 21,60 g (75-%-ige Ausbeute) der Verbindung VIII erhalten. $R_{fD}$ 0,32, $E_{1,2}$ 1,12.

**Schritt 9: BOC-Val-Pro-Pro-OBzl (IX)**

Ausgehend von 13,79 g (63,45 mMol) BOC-Val-OH und 21,5 g (63,45 mMol) HCl · H-Pro-Pro-OBzl (VIII), wobei wie im Schritt 7 beschrieben, verfahren wurde, wurde 22,28 g (70-%-ige Ausbeute der Verbindung IX nach Reinigung durch Säulenchromatographie auf Silicagel (Merck) 0,040 bis 0,063 mm, eluiert mit Ethylacetat : Methanol = 98 : 2, als Öl erhalten.

**Schritt 10: BOC-Val-Pro-Pro-OH (X)**

22,28 g (44,42 mMol) BOC-Val-Pro-Pro-OBzl (IX), aufgelöst in 150 ml Methanol, wurden bei Raumtemperatur und unter atmosphärischem Druck in Gegenwart von 4,46 g 10 Gew.-% Palladium-auf-Holzkohle hydriert. Der Katalysator wurde durch Filtration entfernt und die Lösung im Vakuum konzentriert. Der Rückstand wurde in Ethylacetat aufgelöst und im Vakuum konzentriert. Durch Verdünnung mit Diethylether wurden 10,98 g (60-%-ige Ausbeute) der Verbindung X erhalten: Schmelzpunkt 184 bis 190°C; $[\alpha]^{20}_D$ = -149,9° (c = 1, MeOH); $R_{fB}$ 0,24, $R_{fC}$ 0,53; $E_{5,8}$ 0,51.

**Schritt 11: BOC-Val-Pro-Pro-Leu-Gly-Trp-Met-OMe (XI)**

Ausgehend von 9,10 g (22,12 mMol) BOC-Val-Pro-Pro-OH (X) und 12,30 g (22,12 mMol) HCl · H-Leu-Gly-Trp-Met-OMe (VI), wobei wie im Schritt 5 beschrieben, verfahren wurde, jedoch als Eluierungssystem Ethylacetat verwendet wurde, welches während der chromatischen Reinigung eine von 5 bis 30 % zunehmende Menge un Methanol enthielt, wurden 16,16 g (80-%-ige Ausbeute) der Verbindung XI aus iPrOH/$iPr_2O$ erhalten: Schmelzpunkt 184 bis 190°C; $[\alpha]^{20}_D$ = -98,9° (c = 1, MeOH); $R_{fB}$ 0,13, $R_{fC}$ 0,58.

**Schritt 12: BOC-Val-Pro-Pro-Leu-Gly-Trp-Met-OH (XII)**

5,00 g (5,48 mMol) BOC-Val-Pro-Pro-Leu-Gly-Trp-Met-OMe (XI) wurden in 20 ml Methanol aufgelöst und mit 10 ml 1M Natriumhydroxid während 2 h bei Raumtemperatur verseift.

Die Lösung wurde mit Wasser verdünnt, teilweise im Vakuum konzentriert, auf 0°C abgekühlt, auf pH 2 mit wäßriger 5M Salzsäure angesäuert, und dann mehrere Male mit Ethylacetat extrahiert. Die organische Schicht wurde bis zur Neutralität mit gesättigter wäßriger Natriumchloridlösung gewaschen und über wasserfreiem Natriumsulfat getrocknet. Nach Entfernung des Lösungsmittels wurden 3,99 g (81-%-ige Ausbeute) der Verbindung XII erhalten: Schmelzpunkt 194 bis 200°C (Zersetzung); $[\alpha]^{20}_D$ = -102,9° (c = 1, MeOH); $R_{fC}$ 0,34; $E_{5,8}$ 0,18 Glu.

**Schritt 13: H-Val-Pro-Pro-Leu-Gly-Trp-Met-OH · HCl (XIII)**

Ausgehend von 3,85 g (4,28 mMol) BOC-Val-Pro-Pro-Leu-Gly-Trp-Met-OH (XII), wobei wie im Schritt 6 beschrieben, verfahren wurde, wurden 3,05 g der rohen Verbindung XIII aus iPrOH/$iPr_2O$ erhalten. Das Rohprodukt wurde dann auf DEAE-Sephadex A-25 (Handelsname) gereinigt, wobei als Eluierungsmittel eine 0,02M Ammoniumacetatlösung bei pH 6,7 verwendet wurde. Nach Lyophilisierung aus Essigsäure wurde das Produkt erneut in Essigsäure aufgelöst und mit 6 ml einer gesättigten Lösung an Chlorwasserstoff in Essigsäure behandelt. Die Lösung wurde in Diethylether gegossen.

Auf diese Weise wurden 2,57 g (72-%-ige Ausbeute) der Verbindung XIII erhalten: Schmelzpunkt 150°C; $[\alpha]^{22}_D$ = -90,5° (c = 1, MeOH); $R_{fD}$ 0,30; $E_{1,2}$ 0,58 Glu.

**Beispiel 2**

**Herstellung von HCl · H-Val-Pro-Pro-Leu-Gly-Trp-Met-OMe (XIV)**

Ausgehend von 5,00 g (4,28 mMol) BOC-Val-Pro-Pro-Leu-Gly-Trp-Met-OMe (XI), hergestellt im Beispiel 1, Schritt 11, wobei wie im Beispiel 1, Schritt 6 beschrieben, verfahren wurden, jedoch als Eluierungssystem $CH_2Cl_2$ : MeOH : $H_2O$ = 86 : 14 : 1 zur chromatographischen Reinigung verwendet wurde, wurden 2,73 g (75-%-ige Ausbeute) der Verbindung XIV aus AcOEt erhalten: Schmelzpunkt 154°C; $[\alpha]^{22}_D$ = -92,4° (c = 1, MeOH); $R_{fD}$ 0,34; $E_{1,2}$ 0,58 Glu.

**Beispiel 3**

**Herstellung von HCl · H-Val-Pro-Pro-Leu-Gly-Trp-Met-$NH_2$ (XVI)**

**Schritt 1 : BOC-Val-Pro-Pro-Leu-Gly-Trp-Met-$NH_2$ (XV)**

5,00 g (4,28 mMol) BOC-Val-Pro-Pro-Leu-Gly-Trp-Met-OMe (XI), hergestellt im Beispiel 1, Schritt 11, wurden in einer Lösung von 100 ml Methanol und 2 ml Ethylenglykol, gesättigt mit Ammoniak bei 0°C, aufgelöst. Das Reaktionsgemisch wurde 3 Tage lang in einem Kühlschrank belassen und dann das überschüssige Ammoniak und das Lösungsmittel im Vakuum entfernt. Das Rohprodukt wurde teilweise durch Säulenchromatographie auf Silicagel (Merck) 0,040 bis 0,063 mm gereinigt, wobei zur Eluierung AcOEt : MeOH = 87 : 13 verwendet wurde. Das erhaltene Produkt wurde als solches im nächsten Schritt verwendet (2,58 g der Verbindung XV wurden aus iPrOH/$iPr_2O$ erhalten): $R_{fC}$ = 0,31.

**Schritt 2: H-Val-Pro-Pro-Leu-Gly-Trp-Met-$NH_2$ · HCl (XVI)**

Ausgehend von 2,45 g (2,73 mMol) BOC-Val-Pro-Pro-Leu-Gly-Trp-Met-$NH_2$ (XV), wobei wie im Beispiel 1, Schritt 6 beschrieben, verfahren wurde, jedoch als Eluierungssysteme $CH_2Cl_2$ : MeOH : $H_2O$ = 85 : 15:1 nach dem Volumen und $CH_2Cl_2$ : MeOH : $H_2O$ = 80 : 20 : 1 nach dem Volumen zur chromatographischen Reinigung verwendet wurden, wurden 1,55 g (68-%-ige Ausbeute) der Verbindung XVI aus MeOH/iPrOH/$iPr_2O$ nach Entsalzen auf Sephadex G-10 (Markenname) erhalten: Schmelzpunkt 150 bis 158°C; $[\alpha]^{24}_D$ = -74,8° (c = 1, MeOH); $R_{fD}$ 0,36; $E_{1,2}$ 0,55 Glu.

**Beispiel 4 Aufzuchtversuch bei Schweinen**

Der Aufzuchtversuch wurde über eine Zeitdauer von 4 Wochen durchgeführt, wobei Gruppen von 7 kastrierten männlichen Schweinen (Hybride), die in Untergruppen aufgeteilt und in Käfigen mit flachen Tragflächen gehalten wurden, eingesetzt wurden.

Die Schweine wurden während der ganzen Versuchsdauer mit Aufzuchtfutter (Höveler, Normaltyp ohne Futteradditive), 1 kg pro Tag gefüttert. Wasser wurde ad libitum verabreicht.

Das Gewicht der Schweine wurde wöchentlich aufgezeichnet. Das Versuchsprotokoll wird in der nachfolgenden Tabelle wiedergegeben.

| Gruppe | Anzahl d. Tiere | Behandlung mit | Dosis | Parameters |
|---|---|---|---|---|
| 1 | 2 | - | - | Körpergewicht |
| 2 | 2 | NaCl phys. | 0.1 ml/kc * | Futterverbrauch |
| 3 | 3 | TK-7 (Beisp.1) | 50 ng/kg * | Futterverwertung (ng/Futter/kg Gewichtszunahme) |

* nach dem Gewicht

**Ergebnisse**

| Gruppe | 1 | 2 | 3 |
|---|---|---|---|
| Behandlung | - | NaCl phys. | TK-7 (Beisp.1) |
| Dosis (ng/kg) | - | - | 50 |
| Ausgangsgewicht (kg) | 12,5 | 14,8 | 11,7 |
| Gewicht nach 1 Woche | 14,0 | 16,3 | 15,2 |

| Gruppe | 1 | 2 | 3 |
|---|---|---|---|
| Gewicht nach 2 Wochen | 16,0 | 18,5 | 17,8 |
| Gewicht nach 3 Wochen | 19,3 | 20,5 | 20,7 |
| Gawichtszunahme in kg | 6,8 | 5,7 | 9,0 |
| Gewichtszunahme % | 54,4 | 38,5 | 76,9 |
| Δ% zur Kontrollgruppe | | -16,2 | +32,4 |
| Verbrauch (kg) | 21 | 21 | 21 |
| Futterverwertung | 3,09 | 3,68 | 2,33 |
| Δ% zur Kolntrolgruppe | | +19,1 | -24,6 |

Die tägliche Injektion an physiologischer Kochsalzlösung verursachte eine Abnahme des Gewichtszunahme im Vergleich zu der nicht-behandelten Gruppe. Trotz dieses reduzierten Wachstums, das auf einen Injektionstest zurückzuführen ist, zeigten die Tiere mit täglichen Injektionen an TK-7 eine deutliche Gewichtszunahme. Die Futterverwertung spiegelt die Ergebnisse der Gewichtszunahme wieder.

**Patentansprüche** für die Vertragsstaaten: BE, CH, DE, FR, GB, IT, LI, NL, SE.

1. Heptapeptid der Formel:

X-Val-Pro-Pro-Leu-Gly-Trp-A-Y,

worin bedeuten:

X ein Wasserstoffatom oder eine terminale N-Schutzgruppe vom Acyl-, aliphatischen Urethan-, aromatischen Urethan-, Alkyl- oder Aralkyltyp; A ein neutraler L-α-Aminosäurerest; und Y eine Hydroxygruppe, eine Aminogruppe oder eine Gruppe der Formel OR, NHR, $NR_2$ oder NH-NH-R', worin R eine geradkettige, verzweigte oder cyclische (einschließlich kondensierte oder Brückenring-) Alkylgruppe mit bis zu 11 Kohlenstoffatomen bedeutet, welche nichtsubstituiert oder mit einer Hydroxy-oder Aminogruppe oder einem Halogenatom substituiert ist; eine Aralkylgruppe mit 7 bis 14 Kohlenstoffatomen oder eine Phenylgruppe; und R' ein Wasserstoffatom, eine beliebige Gruppe, wie sie für R definiert ist, eine geradkettige, verzweigte oder cyclische aliphatische Acylgruppe mit 1 bis 11 Kohlenstoffatomen, die nichtsubstituiert oder mit einer Hydroxy- oder einer Aminogruppe oder einem Halogenatom substituiert ist, eine aromatische Acylgruppe, die nichtsubstituiert oder mit einer Hydroxy- oder Aminogruppe oder einem Halogenatom substituiert ist, eine geradkettige, verzweigte oder cyclische aliphatische Urethangruppe mit 3 bis 11 Kohlenstoffatomen, oder eine aromatische Gruppe vom Urethangruppe bedeutet, oder ein pharmazeutisch oder tierarzneilich annehmbares Salz desselben.

2. Peptid gemäß Anspruch 1 worin X ein Wasserstoffatom oder ein Formyl, Acetyl, Trifluoracetyl, Propionyl, Benzoyl, Benzyloxycarbonyl, 4-Nitrobenzyloxycarbonyl, 4-Methoxybenzyloxycarbonyl, 2,4-Dichlorbenzyloxycarbonyl, 2-Bromobenzyloxycarbonyl, 9-Fluorenylmethoxy-carbonyl und 3,5-Dimethoxy-α,α'-dimethylbenzyloxy-carbonyl, t-Butoxycarbonyl, 1-Methyl-cyclobutoxy-carbonyl, Adamantyloxycarbonyl, Isobornyloxycarbonyl, Methylsulfonylethoxycarbonyl, Trityl, Benzyl, Methyl oder Isopropyl bedeutet, A einen Rest Met, Nle,Ile, Leu oder Phe darstellt; Y eine Hydroxygruppe, eine Aminogruppe oder eine Gruppe der Formel OR, NHR, $NR_2$ oder NH-NH-R' bedeutet, worin R ein Methyl, Ethyl, n-Propyl, Isopropyl, n-Butyl, s-Butyl, Isobutyl, t-Butyl, 2,2,2-Trifluorethyl, Cyclohexyl, Adamantyl, Phenyl, Benzyl, Fluorenylmethyl oder eine Phenethylgruppe darstellt, R' ein Wasserstoffatom, eine beliebige der Gruppen, wie sie in diesem Anspruch speziell für R genannt sind, oder ein Formyl, Acetyl, Trifluoracetyl, Propionyl, Butyryl, Adamantylcarbonyl, Benzoyl, Phenylacetyl, Cinnamyl, Benzyloxycarbonyl, 4-Nitrobenzyloxycarbonyl, 4-Methoxybenzyloxycarbonyl, 2,4-Dichlorbenzyloxycarbonyl, 2-Brombenzyl-oxycarbonyl, 9-Fluorenylmethoxycarbonyl, 3,5-Dimethoxy-α,α'-dimethylbenzyloxycarbonyl, t-Butoxycarbonyl, 1-Methyl-cyclobutoxycarbonyl, Adamantyloxycarbonyl, Methylsulfonylethoxycarbonyl oder Isobornyloxycarbonyl bedeutet; oder ein Säure- oder Basenadditionssalz desselben, wobei das genannte Säureadditionssalz abgeleitet ist von Schwefelsäure, Phosphorsäure, Salzsäure, Bromwasserstoffsäure, Jodwasserstoffsäure, Salpetersäure, Sulfaminsäure, Zitronensäure, Milchsäure, Brenztraubensäure, Oxalsäure, Maleinsäure, Bernsteinsäure, Weinsäure, Zimtsäure, Essigsäure, Trifluoressigsäure, Benzoesäure, Salicylsäure, Gluconsäure, Ascorbinsäure und das genannte Basenadditionssalz von Natriumhydroxid, Kaliumhydroxid, Diethylamin, Triethylamin oder Dicyclohexylamin abgeleitet ist.

3. Peptid gemäß Anspruch 2, dadurch *gekennzeichnet*, daß X ein Wasserstoffatom darstellt.

4. Peptid gemäß Anspruch 3, dadurch *gekennzeichnet*, daß A einen Met-Rest bedeutet.

5. Peptid der Formel

H-Val-Pro-Pro-Leu-Gly-Trp-Met-OMe

oder ein pharmazeutisch oder tierarzneilich annehmbares Salz desselben.

6. Peptid der Formel

H-Val-Pro-Pro-Leu-Gly-Trp-Met-OH

oder ein pharmazeutisch oder tierarzneilich annehmbares Salz desselben.

7. Peptide gemäß den folgenden Formeln:

H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-Ome
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

oder ein pharmazeutisch oder tierarzneilich annehmbares Salz derselben.

8. Verfahren zur Förderung der Wachstumsrate bei Tieren, dadurch *gekennzeichnet*, daß man täglich in den Futterrationen oder durch subkutane Implantierung eine wirksame Menge eines Peptides gemäß Anspruch 1 oder eines tiermedizinisch annehmbaren Salzes desselben verabreicht.

9. Tierisches Futter, dadurch *gekennzeichnet*, daß demselben ein Peptid gemäß Anspruch 1 oder ein tierarzneilich annehmbares Salz desselben zugemischt ist.

10. Veterinäre Zubereitung, *gekennzeichnet* durch ein Peptid gemäß Anspruch 1 oder ein tiermedizinisch annehmbares Salz desselben im Gemisch mit einem tiermedizinisch annehmbaren Verdünner oder Träger.

11. Verfahren zur Herstellung einer Verbindung gemäß Anspruch 1 *gekennzeichnet* durch Kondensieren, gegebenenfalls in Gegenwart von Dicyclohexylcarbodiimid, einer Verbindung der Formel II

X-Val-Pro-Pro-OH II

oder eines gemischten Anhydrides, aktivierten Esters oder Azids desselben mit einer Verbindung der allgemeinen Formel III

H-Leu-Gly-Trp-A-Y III,

worin X, A und Y die im Anspruch 1 angegebenen Bedeutungen haben, mit der Ausnahme, daß X nicht Wasserstoff und Y nicht $NHNH_2$ darstellt, und - sofern dies gewünscht wird - Umsetzung des erhaltenen Peptids gemäß Anspruch 1 in einem oder mehreren weiteren Schritten zur

(a) Umwandlung der Gruppe Y in eine andere Gruppe Y durch Hydrazinolyse, Veresterung, Hydrolyse oder Ammonolyse,
(b) Entfernung der Schutzgruppe am Peptid,
(c) Salzbildung am freien Peptid und
(d) Erhalten eines freien Peptides aus einem entsprechenden Salz.

**Patentansprüche** für den Vertragsstaat: AT

1. Verfahren zur Herstellung eines Heptapeptids der Formel

X-Val-Pro-Pro-Leu-Gly-Trp-A-Y,

worin bedeuten: X ein Wasserstoffatom oder eine terminale N-Schutzgruppe vom Acyl-, aliphatischen Urethan-, aromatischen Urethan-, Alkyl- oder Aralkyltyp;

A ein neutraler L-α-Aminosäurerest; und Y eine Hydroxygruppe, eine Aminogruppe oder eine Gruppe der Formel OR, NHR, $NR_2$ oder NH-NH-R', worin R eine geradkettige, verzweigte oder cyclische (einschließlich kondensierte oder Brückenring-) Alkylgruppe mit bis zu 11 Kohlenstoffatomen bedeutet, welche nichtsubstituiert oder mit einer Hydroxy- oder Aminogruppe oder einem Halogenatom substituiert ist; eine Aralkylgruppe mit 7 bis 14 Kohlenstoffatomen oder eine Phenylgruppe; und R' ein Wasserstoffatom, eine beliebige Gruppe, wie sie für R definiert ist, eine geradkettige, verzweigte oder cyclische aliphatische Acylgruppe mit 1 bis 11 Kohlenstoffatomen, die nichtsubstituiert oder mit einer Hydroxy- oder einer Aminogruppe oder einem Halogenatom substituiert ist, eine aromatische Acylgruppe, die nichtsubstituiert oder mit einer Hydroxy- oder Aminogruppe oder einem Halogenatom substituiert ist, eine geradkettige, verzweigte oder cyclische aliphatische Urethangruppe mit 3 bis 11 Kohlenstoffatomen, oder eine aromatische Gruppe vom Urethangruppe bedeutet, oder eines pharmazeutisch oder tieranrzneilich annehmbaren Salzes desselben, *gekennzeichnet* durch

Kondensieren, gegebenenfalls in Gegenwart von Dicylohexylcarbodiimid, einer Verbindung der Formel II

X-Val-Pro-Pro-OH II

oder eines gemischten Anhydrides, aktivierten Esters oder Azids desselben mit einer Verbindung der allgemeinen Formel III

H-Leu-Gly-Trp-A-Y III,

worin X, A und Y die im Anspruch 1 angegebenen Bedeutungen haben, mit der Ausnahme, daß X nicht Wasserstoff und Y nicht $NHNH_2$ darstellt, und - sofern dies gewünscht wird - Umsetzung des erhaltenen Peptids gemäß Anspruch 1 in einem oder mehreren weiteren Schritten zur

(a) Umwandlung der Gruppe Y in eine andere Gruppe Y durch Hydrazinolyse, Veresterung, Hydrolyse oder Ammonolyse,
(b) Entfernung der Schutzgruppe am Peptid,
(c) Salzbildung am freien Peptid und
(d) Erhalten eines freien Peptides aus einem entsprechenden Salz.

2. Verfahren gemäß Anspruch 1 dadurch *gekennzeichnet*, daß ein Heptapeptid hergestellt wird, wie dies im Anspruch 1 definiert ist, worin X ein Wasserstoffatom, oder ein Formyl, Acetyl, Trifluoracetyl, Propionyl, Benzoyl, Benzyloxycarbonyl, 4-Nitrobenzyloxycarbonyl, 4-Methoxybenzyloxycarbonyl, 2,4-Dichlorbenzyloxycarbonyl, 2-Bromobenzyloxycarbonyl, 9-Fluorenylmethoxy-carbonyl und 3,5-Dimethoxy-α,α'-dimethylbenzyloxy-carbonyl, t-Butoxycarbonyl, 1-Methyl-cyclobutoxycarbonyl, Adamantyloxycarbonyl, Isobornyloxycarbonyl, Methylsulfonylethoxycarbonyl, Trityl, Benzyl, Methyl oder Isopropyl bedeutet, A einen Rest Met, Nle,Ile, Leu oder Phe darstellt; Y eine Hydroxygruppe, eine Aminogruppe oder eine Gruppe der Formel OR, NHR, $NR_2$ oder NH-NH-R' bedeutet, worin R ein Methyl, Ethyl, n-Propyl, Isopropyl, n-Butyl, s-Butyl, Isobutyl, t-Butyl, 2,2,2-Trifluorethyl, Cyclohexyl, Adamantyl, Phenyl, Benzyl, Fluorenylmethyl oder eine Phenethylgruppe darstellt, R' ein Wasserstoffatom, eine beliebige der Gruppen, wie sie in diesem Anspruch speziell für R genannt sind, oder ein Formyl, Acetyl, Trifluoracetyl, Propionyl, Butyryl, Adamantylcarbonyl, Benzoyl, Phenylacetyl, Cinnamyl, Benzyloxycarbonyl, 4-Nitrobenzyloxycarbonyl, 4-Methoxybenzyloxycarbonyl, 2,4-Dichlorbenzyloxycarbonyl, 2-Brombenzyl-oxycarbonyl, 9-Fluorenylmethoxycarbonyl, 3,5-Dimethoxy-α,α'-dimethylbenzyloxycarbonyl, t-Butoxycarbonyl, 1-Methyl-cyclobutoxycarbonyl, Adamantyloxycarbonyl, Methylsulfonylethoxycarbonyl oder Isobornyloxycarbonyl bedeutet; oder ein Säure- oder Basenadditionssalz desselben, wobei das genannte Säureadditionssalz abgeleitet ist von Schwefelsäure, Phosphorsäure, Salzsäure, Bromwasserstoffsäure, Jodwasserstoffsäure, Salpetersäure, Sulfaminsäure, Zitronensäure, Milchsäure, Brenztraubensäure, Oxalsäure, Maleinsäure, Bernsteinsäure, Weinsäure, Zimtsäure, Essigsäure, Trifluoressigsäure, Benzoesäure, Salicylsäure, Gluconsäure, Ascorbinsäure und das genannte Basenadditionssalz von Natriumhydroxid, Kaliumhydroxid, Diethylamin, Triethylamin oder Dicyclohexylamin abgeleitet ist.

3. Verfahren gemäß Anspruch 2, dadurch *gekennzeichnet*, daß X ein Wasserstoffatom darstellt.

4. Verfahren gemäß Anspruch 3, dadurch *gekennzeichnet*, daß A einen Met-Rest bedeutet.

5. Verfahren gemäß Anspruch 1, dadurch *gekennzeichnet*, daß das Peptid die folgende Formel besitzt:

H-Val-Pro-Pro-Leu-Gly-Trp-Met-OMe

oder eine pharmazeutisch oder tierarzneilich annehmbares Salz desselben darstellt.

6. Verfahren gemäß Anspruch 1 dadurch *gekennzeichnet*, daß das Peptid die folgende Formel besitzt:

H-Val-Pro-Pro-Leu-Gly-Trp-Met-OH

oder ein pharmazeutisch oder tierarzneilich annehmbares Salz desselben darstellt.

7. Verfahren gemäß Anspruch 1, dadurch *gekennzeichnet*, daß die Peptide den folgenden Formeln entsprechen:

H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-Ome
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

oder pharmazeutisch oder tierarzneilich annehmbare Salze derselben darstellen.

8. Verfahren zur Förderung der Wachstumsrate bei Tieren, dadurch *gekennzeichnet*, daß man täglich in den Futterrationen oder durch subkutane Implantierung eine wirksame Menge eines Peptides gemäß Anspruch 1 oder eines tiermedizinisch annehmbaren Salzes desselben, verabreicht.

9. Verfahren zur Herstellung eines tierischen Futters, dadurch *gekennzeichnet*, daß man dem Futter ein Heptapeptid, wie es im Anspruch 1 definiert ist oder ein tierarzneilich annehmbares Salz desselben zumischt.

10. Verfahren zur Herstellung einer veterinären Zubereitung, dadurch *gekennzeichnet*, daß man ein Heptapeptid, wie es im Anspruch 1 definiert ist, oder ein tiermedizinisch annehmbares Salz desselben mit einem tiermedizinisch annehmbaren Verdünner oder Träger vermischt.

**Claims** for the contracting states: BE, CH, DE, FR, GB, IT, LI, NL, SE

1. Heptapeptide of the formula:

X-Val-Pro-Pro-Leu-Gly-Trp-A-Y,

where
X stands for a hydrogen atom or a terminal nitrogen protecting group of the acyl, aliphatic urethane, aromatic urethane, alkyl or aralkyl type;
A stands for a neutral L-α-aminoacid group; and
Y stands for a hydroxyl group, an amino group or a group of the formula OR, NHR, $NR_2$ or NH-NH-R', where R stands for a straightchain, branched or cyclic (including condensed or bridged ring) alkyl group with up to 11 carbon atoms, which is unsubstituted or substituted with a hydroxyl or amino group or a halogen atom, an aralkyl group with 7 to 14 carbon atoms or a phenyl group; and R stands for a hydrogen atom, any group defined like R, a straightchain, branched or cyclic aliphatic acyl group with 1 to 11 carbon atoms, which is unsubstituted or substituted with a hydroxyl or amino group or a halogen atom, an aromatic acyl group, which is unsubstituted or substituted with a hydroxyl or amino group or a halogen atom, a straight-chain, branched or cyclic aliphatic urethane group with 3 to 11 carbon atoms, or an aromatic urethane group,

or a salt thereof which is acceptable pharmaceutically or in veterinary pharmacology.

2. Peptide in accordance with claim 1, where X stands for a hydrogen atom or a formyl, acetyl, trifluoroacetyl, propionyl, benzoyl, benzyloxycarbonyl, 4-nitrobenzyloxycarbonyl, 4-methoxybenzyloxycarbonyl, 2,4-dichlorobenzyloxycarbonyl, 2-bromobenzyloxycarbonyl, 9-fluorenylmethoxycarbonyl and 3,5-dimethoxy-α-α'-dimethylbenzyloxycarbonyl, t-butoxycarbonyl, 1-methylcyclobutoxycarbonyl, adamantyloxycarbonyl, isobornyloxycarbonyl, methylsulphonylethoxycarbonyl, trityl,

benzyl, methyl or isopropyl;

A represents a Met, Nle, Ile, Leu or Phe group; [and] Y stands for a hydroxyl group, an amino group or a group of the formula OR, NHR, $NR_2$ or NH-NH-R, where R represents a methyl, ethyl, n-propyl, isopropyl, n-butyl, s-butyl, isobutyl, t-butyl, 2,2,2-trifluoroethyl, cyclohexyl, adamantyl, phenyl, benzyl, fluorenylmethyl or a phenethyl group, [and] R' stands for a hydrogen atom, any one of the groups specially mentioned in this claim for R, or a formyl, acetyl, trifluoroacetyl, propionyl, butyryl, adamantylcarbonyl, benzoyl, phenylacetyl, cinnamyl, benzyloxycarbonyl, 4-nitrobenzyloxycarbonyl, 4-methoxybenzyloxycarbonyl, 2,4-dichlorbenzyloxycarbonyl, 2-bromobenzyl-oxycarbonyl, 9-fluorenylmethoxycarbonyl, 3,5-dimethoxy-α,α'-dimethylbenzyloxycarbonyl, t-butoxycarbonyl, 1-methylcyclobutoxycarbonyl, adamantyloxycarbonyl, methylsulphonylethoxycarbonyl or isobornyloxycarbonyl; or an acid or base addition salt of the same, the said acid addition salt being derived from sulphuric acid, phosphoric acid, hydrochloric acid, hydrobromic acid, hydriodic acid, nitric acid, sulphamic acid, citric acid, lactic acid, pyruvic acid, oxalic acid, maleic acid, succinic acid, tartaric acid, cinnamic acid, acetic acid, trifluoroacetic acid, benzoic acid, salicylic acid, gluconic acid [or] ascorbic acid, and the said base addition salt being derived from sodium hydroxide, potassium hydroxide, diethylamine, triethylamine or dicyclohexylamine.

3. Peptide according to claim 2, *characterized* in that X represents a hydrogen atom.

4. Peptide according to claim 3, *characterized* in that A stands for a Met group.

5. Peptide of the formula

H-Val-Pro-Pro-Leu-Gly-Trp-Met-OMe

or a salt of the same which is acceptable pharmaceutically or in veterinary pharmacology.

6. Peptide of the formula

H-Val-Pro-Pro-Leu-Gly-Trp-Met-OH

or a salt of the same which is acceptable pharmaceutically or in veterinary pharmacology.

7. Peptides in accordance with the following formulae:
H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-OMe
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

or a salt of the same which is acceptable pharmaceutically or in veterinary pharmacology.

8. Process for the promotion of the growth rate of animals, *characterized* in that an effective amount of a peptide in accordance with Claim 1 or of a salt thereof which is acceptable in veterinary medicine is administered daily in the feed rations or by subcutaneous implantation.

9. Animal feed, *characterized* in that a peptide in accordance with Claim 1 or a salt thereof which is acceptable in veterinary pharmacology is admixed with it.

10. Veterinary preparation, *characterized* by a peptide in accordance with Claim 1 or a salt thereof which is acceptable in veterinary medicine mixed with a diluent or carrier which is acceptable in veterinary medicine.

11. Process for production of a compound according to Claim 1, *characterized* by condensation, possibly in presence of dicyclohexylcarbodiimide, of a compound of formula II

X-Val-Pro-Pro-OH II

or a mixed anhydride, activated ester or azide thereof with a compound of the general formula III

H-Leu-Gly-Trp-A-Y III,

in which X, A and Y have the meanings given in claim 1, except that X does not represent hydrogen and Y does not represent $NHNH_2$, and - provided this is desired - reaction in one or more further steps of the peptide according to Claim 1 which is obtained, for

(a) conversion of the group Y to another group Y by hydrazinolysis, esterification, hydrolysis or ammonolysis,
(b) removal of the protecting group on the peptide,
(c) formation of a salt on the free peptide and
(d) obtaining a free peptide from a corresponding salt.

**Claims** the contracting state: AT

1. Process for the production of a heptapeptide of the formula:

X-Val-Pro-Pro-Leu-Gly-Trp-A-Y,

where
X stands for a hydrogen atom or a terminal nitrogen protecting group of the acyl, aliphatic urethane, aromatic urethane, alkyl or aralkyl type;
A stands for a neutral L-α-aminoacid group; and
Y stands for a hydroxyl group, an amino group or a group of the formula OR, NHR, $NR_2$ or NH-NH-R', where R stands for a straightchain, branched or cyclic (including condensed or bridged ring) alkyl group with up to 11 carbon atoms, which is unsubstituted or substituted with a hydroxyl or amino group or a halogen atom; an aralkyl group with 7 to 14 carbon atoms or a phenyl group; and R' stands for a hydrogen atom, any group defined like R, a straightchain, branched or cyclic aliphatic acyl group with 1 to 11 carbon atoms, which is unsubstituted or substituted with a hydroxyl or amino group or a halogen atom, an aromatic acyl group, which is unsubstituted or substituted with a hydroxyl or amino group or a halogen atom, a straight-chain, branched or cyclic aliphatic urethane group with 3 to 11 carbon atoms, or an aromatic urethane group, or a salt thereof which is acceptable pharmaceutically or in veterinary pharmacology, *characterized* by condensing, possibly in presence of dicyclohexylcarbodiimide, a compound of formula II

X-Val-Pro-Pro-OH II

or a mixed anhydride, activated ester or azide thereof with a compound of the general formula III

H-Leu-Gly-Trp-A-Y III,

in which X, A and Y have the meanings given in claim 1, except that X does not represent hydrogen and Y does not represent $NHNH_2$, and - provided this is desired - reaction in one or more further steps of the peptide according to Claim 1 which is obtained, for

(a) conversion of the group Y to another group Y by hydrazinolysis, esterification, hydrolysis or ammonolysis,
(b) removal of the protecting group on the peptide,
(c) formation of a salt on the free peptide and
(d) obtaining a free peptide from a corresponding salt.

2. Process in accordance with claim 1, *characterized* in that a heptapeptide as defined in claim 1 is produced, in which
X stands for a hydrogen atom or a formyl, acetyl, trifluoroacetyl, propionyl, benzoyl, benzyloxycarbonyl, 4-nitrobenzyloxycarbonyl, 4-methoxybenzyloxycarbonyl, 2, 4-dichlorobenzyloxycarbonyl, 2-bromobenzyloxycarbonyl, 9-fluorenylmethoxycarbonyl and 3,5-dimethoxy-α-α'-dimethylbenzyloxycarbonyl, t-butoxycarbonyl, 1-methylcyclobutoxycarbonyl, adamantyloxycarbonyl, isobornyloxycarbonyl, methylsulphonylethoxycarbonyl, trityl, benzyl, methyl or isopropyl;
A represents a Met, Nle, Ile, Leu or Phe group; [and]
Y stands for a hydroxyl group, an amino group or a group of the formula OR, NHR, $NR_2$ or NH-NH-R', where
R represents a methyl, ethyl, n-propyl, isopropyl, n-butyl, s-butyl, isobutyl, t-butyl, 2,2,2-trifluoroethyl, cyclohexyl, adamantyl, phenyl, benzyl, fluorenylmethyl or a phenethyl group, [and]
R' stands for a hydrogen atom, any one of the groups specially mentioned in this claim for R, or a formyl, acetyl, trifluoroacetyl, propionyl, butyryl, adamantylcarbonyl, benzoyl, phenylacetyl, cinnamyl, benzyloxycarbonyl, 4-nitrobenzyloxycarbonyl, 4-methoxybenzyloxycarbonyl, 2,4-dichlorobenzyloxycarbonyl, 2-bromobenzyl-oxycarbonyl, 9-fluorenylmethoxycarbonyl, 3,5-dimethoxy-α,α'-dimethylbenzyloxycarbonyl, t-butoxycarbonyl, 1-methyl-cyclobutoxycarbonyl, adamantyloxycarbonyl, methylsulphonylethoxycarbonyl or isobornyloxycarbonyl; or an acid or base addition salt of the same, the said acid addition salt being derived from sulphuric acid, phosphoric acid, hydrochloric acid, hydrobromic acid, hydriodic acid, nitric acid, sulphamic acid, citric acid, lactic acid, pyruvic acid, oxalic acid, maleic acid, succinic acid, tartaric acid, cinnamic acid, acetic acid, trifluoroacetic acid, benzoic acid, salicylic acid, gluconic acid [or] ascorbic acid, and the said

base addition salt being derived from sodium hydroxide, potassium hydroxide, diethylamine, triethylamine or dicyclohexylamine.

3. Process according to claim 2, *characterized* in that X represents a hydrogen atom.

4. Process according to claim 3, *characterized* in that A stands for a Met group.

5. Process according to claim 1, *characterized* in that the peptide has the following formula:

H-Val-Pro-Pro-Leu-Gly-Trp-Met-OMe

or a salt of the same which is acceptable pharmaceutically or in veterinary pharmacology.

6. Process according to claim 1, *characterized* in that the peptide has the following formula:

H-Val-Pro-Pro-Leu-Gly-Trp-Met-OH

or a salt of the same which is acceptable pharmaceutically or in veterinary pharmacology.

7. Process according to claim 1 *characterized* in that the peptides correspond to the following formulae:

H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-OMe
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

or salts of the same which are acceptable pharmaceutically or in veterinary pharmacology.

8. Process for the promotion of the growth rate of animals, *characterized* in that an effective amount of a peptide in accordance with claim 1 or of a salt thereof which is acceptable in veterinary medicine is administered daily in the feed rations or by subcutaneous implantation.

9. Process for the production of an animal feed, *characterized* in that a heptapeptide as defined in claim 1 or a salt thereof which is acceptable in veterinary pharmacology is admixed with the feed.

10. Process for production of a veterinary preparation, *characterized* in that a heptapeptide as defined in claim 1 or a salt thereof which is acceptable in veterinary medicine is mixed with a diluent or carrier which is acceptable in veterinary medicine.

**Revendications** pour les Etats Contractants: BE, CH, DE, FR, GB, IT, LI, NL, SE.

1. Heptapeptide représenté par la formule:

X-Val-Pro-Pro-Leu-Gly-Trp-A-Y,

dans laquelle :

- représente un atome d'hydrogène ou un groupe de protection du N terminal du type acyle, uréthanne aliphatique, uréthanne aromatique, alkyle ou aralkyle;
- A représente un reste d'acide α-aminé L neutre; et
- Y représente un groupe hydroxy, un groupe amino ou un groupe de formule OR, NHR, $NR_2$ ou NH-NH-R, où:
- R représente un groupe alkyle linéaire, ramifié ou cyclique (y compris à cycles condensés ou à cycles pontés), ayant jusqu'à 11 atomes de carbone, lequel n'est pas substitué ou bien qui est substitué par un groupe hydroxy ou amino ou par un atome d'halogène; un groupe aralkyle ayant de 7 à 14 atomes de carbone ou un groupe phényle; et
- R' représente un atome d'hydrogène, un groupe quelconque choisi parmi ceux entrant dans la définition de R, un

groupe acyle aliphatique, linéaire, ramifié ou cyclique, ayant de 1 à 11 atomes de carbone, qui n'est pas substitué ou bien qui est substitué par un groupe hydroxy ou amino ou par un atome d'halogène, un groupe acyle aromatique, qui n'est pas substitué ou qui est substitué par un groupe hydroxy ou amino ou par un atome d'halogène, un groupe uréthanne aliphatique, linéaire, ramifié ou cyclique, ayant de 3 à 11 atomes de carbone, ou un groupe uréthanne aromatique,

ou l'un de ses sels acceptables du point de vue pharmaceutique ou vétérinaire.

2. Peptide selon la revendication 1, dans lequel :

- X représente un atome d'hydrogène ou un groupe formyle, acétyle, trifluoroacétyle, propionyle, benzoyle, benzyloxycarbonyle , nitro-4 benzyloxycarbonyle, méthoxy-4 benzyloxycarbonyle, dichloro-2,4 benzyloxycarbonyle, bromo-2 benzyloxycarbonyle, fluorényl-9 méthoxy-carbonyle et diméthoxy-3,5 $\alpha,\alpha'$-diméthylbenzyloxycarbonyle, t-butoxycarbonyle, méthyl-1 cyclobutoxycarbonyle, adamantyloxycarbonyle, isobornyloxycarbonyle, méthylsulfonyléthoxycarbonyle, trityle, benzyle, méthyle ou isopropyle ;
- A représente un reste Met, Nle, Ile, Leu ou Phe;
- Y représente un groupe hydroxy, un groupe amino ou un groupe de formule OR, NHR, $NR_2$ ou NH-NH-R', où
- R représente un groupe méthyle, éthyle, n-propyle, isopropyle, n-butyle, s-butyle, isobutyle, t-butyle, trifluoro-2,2,2 éthyle, cyclohexyle, adamantyle, phényle, benzyle, fluorénylméthyle ou un groupe phénétyle ;
- R' représente un atome d'hydrogène, l'un quelconque des groupes tels qu'ils sont cités dans cette revendication spécifiquement pour R, ou un groupe formyle, acétyle, trifluoroacétyle, propionyle, butyryle, adamantylcarbonyle, benzoyle, phénylacétyle, cinnamyle, benzyloxycarbonyle, nitro-4 benzyloxycarbonyle, méthoxy-4 benzyloxycarbonyle, dichloro-2,4 benzyloxycarbonyle, bromo-2 benzyloxycarbonyle, fluorényl-9 méthoxycarbonyle et diméthoxy-3,5 $\alpha,\alpha'$-diméthylbenzyloxycarbonyle, t-butoxycarbonyle, méthyl-1 cyclobutoxycarbonyle, adamantyloxycarbonyle, méthylsulfonyléthoxycarbonyle ou isobornyloxycarbonyle; ou l'un de ses sels d'addition avec les acides ou les bases, ledit sel d'addition avec les acides provenant de l'acide sulfurique, l'acide phosphorique, l'acide chlorhydrique, l'acide bromhydrique, l'acide iodhydrique, l'acide nitrique, l'acide sulfamique, l'acide citrique, l'acide lactique, l'acide pyruvique, l'acide oxalique, l'acide maléique, l'acide succinique, l'acide tartrique, l'acide cinnamique, l'acide acétique, l'acide trifluoroacétique, l'acide benzoïque, l'acide salicylique, l'acide gluconique, l'acide ascorbique, et ledit sel d'addition avec les bases provenant de l'hydroxyde de sodium, l'hyroxyde de potassium, la diéthylamine, la triéthylamine ou la dicyclohexylamine.

3. Peptide selon la revendication 2, *caractérisé* par le fait que X représente un atome d'hydrogène.

4. Peptide selon la revendication 3, *caractérisé* par le fait que A représente un reste Met.

5. Peptide représenté par la formule:

H-Val-Pro-Pro-Leu-Gly-Trp-Met-OMe
ou l'un de ses sels acceptables du point de vue pharmaceutique ou vétérinaire.

6. Peptide représenté par la formule:

H-Val-Pro-Pro-Leu-Gly-Trp-Met-OH

ou l'un de ses sels acceptables du point de vue pharmaceutique ou vétérinaire.

7. Peptides représentés par les formules suivantes:

H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-OMe
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

ou leurs sels acceptables du point de vue pharmaceutique ou vétérinaire.

8. Procédé pour favoriser le taux de croissance chez les animaux, *caractérisée* par le fait qu'on administre quotidiennement, dans les rations alimentaires ou par implantation sous-cutanée, une quantité efficace d'un peptide tel que défini à la revendication 1, ou d'un de ses sels acceptables du point de vue vétérinaire.

9. Aliment pour animaux, *caractérisée* par le fait que s'y trouve incorporé un peptide tel que défini à la revendication 1 ou l'un de ses sels acceptables du point de vue vétérinaire.

10. Préparation vétérinaire, *caractérisée* par le fait qu'elle consiste en un peptide tel que défini à la revendication 1 ou l'un de ses sels acceptables du point de vue vétérinaire, en mélange avec un diluant ou support acceptable du point de vue vétérinaire.

11. Procédé de fabrication d'un composé tel que défini à la revendication 1, *caractérisée* par le fait qu'on condense, le cas échéant en présence de dicyclohexylcarbodiimide, un composé représenté par la formule (II):

X-Val-Pro-Pro-OH (II)

ou un anhydride mixte, un ester activé ou un azide de ce composé, avec un composé représenté par la formule générale (III) :

H-Leu-Gly-Trp-A-Y (III),

où X, A et Y ont les significations indiquées à la revendication 1, à l'exception que X ne représente pas hydrogène et que Y ne représente pas $NHNH_2$' et - pour autant qu'on le souhaite - on transforme le peptide obtenu selon la revendication 1 en une ou plusieurs autres étapes en vue de:

(a) la conversion du groupe Y en un autre groupe Y, par hydrazinolyse, estérification, hydrolyse ou ammoniolyse,
(b) l'élimination du groupe protecteur sur le peptide,
(c) la salification sur le peptide libre, et
(d) l'obtention d'un peptide libre à partir d'un sel correspondant.

**Revendication** pour l'Etat Contractant : AT

1. Procédé de fabrication d'un heptapeptide représenté par la formule:

X-Val-Pro-Pro-Leu-Gly-Trp-A-Y,

dans laquelle:
- X représente un atome d'hydrogène ou un groupe de protection du N terminal du type acyle, uréthanne aliphatique, uréthanne aromatique, alkyle ou aralkyle;
- A représente un reste d'acide α-aminé L neutre ; et
- Y représente un groupe hydroxy, un groupe amino ou un groupe de formule OR, NHR, $NR_2$ ou NH-NH-R', où:
- R représente un groupe alkyle linéaire, ramifié ou cyclique (y compris à cycles condensés ou à cycles pontés), ayant jusqu'à 11 atomes de carbone, lequel n'est pas substitué ou bien qui est substitué par un groupe hydroxy ou amino ou par un atome d'halogène; un groupe aralkyle ayant de 7 à 14 atomes de carbone ou un groupe phényle; et
- R' représente un atome d'hydrogène, un groupe quelconque choisi parmi ceux entrant dans la définition de R, un groupe acyle aliphatique, linéaire, ramifié ou cyclique, ayant de 1 à 11 atomes de carbone, qui n'est pas substitué ou bien qui est substitué par un groupe hydroxy ou un groupe amino ou par un atome d'halogène, un groupe acyle aromatique, qui n'est pas substitué ou qui est substitué par un groupe hydroxy ou amino ou par un atome d'halogène, un groupe uréthanne aliphatique, linéaire, ramifié ou cyclique, ayant de 3 à 11 atomes de carbone, ou un groupe uréthanne aromatique,

ou de l'un de ses sels acceptables du point de vue pharmaceutique ou vétérinaire, *caractérisé* par le fait qu'on condense, le cas échéant en présence de dicyclohexylcarbodiimide, un composé représenté par la formule (II):

X-Val-Pro-Pro-OH (II)

ou un anhydride mixte, un ester activé ou un azide de ce composé, avec un composé représenté par la formule générale (III):

H-Leu-Gly-Trp-A-Y (III),

où X, A et Y ont les significations indiquées ci-dessus, à l'exception que X ne représente pas hydrogène et que Y ne représente pas $NHNH_2$' et - pour autant qu'on le souhaite - on transforme le peptide obtenu ci-dessus en une ou

plusieurs autres étapes en vue de:

(a) la conversion du groupe Y en un autre groupe Y, par hydrazinolyse, estérification, hydrolyse ou ammoniolyse,
(b) l'élimination du groupe protecteur sur le peptide,
(c) la salification sur le peptide libre, et
(d) l'obtention d'un peptide libre à partir d'un sel correspondant.

2. Procédé selon la revendication 1, *caractérisé* par le fait qu'on fabrique un heptapeptide tel que défini à la revendication 1, dans lequel:

- X représente un atome d'hydrogène ou un groupe formyle, acétyle, trifluoroacétyle, propionyle, benzoyle, benzyloxycarbonyle, nitro-4 benzyloxycarbonyle, méthoxy-4 benzyloxycarbonyle, dichloro-2,4 benzyloxycarbonyle, bromo-2 benzyloxycarbonyle, fluorényl-9 méthoxycarbonyle et diméthoxy-3,5 α,α'-diméthylbenzyloxycarbonyle, t-butoxycarbonyle, méthyl-1 cyclobutoxycarbonyle, adamantyloxycarbonyle, isobornyloxycarbonyle, méthylsulfonyléthoxycarbonyle, trityle, benzyle, méthyle ou isopropyle;
- A représente un reste Met, Nle, Ile, Leu ou Phe;
- Y représente un groupe hydroxy, un groupe amino ou un groupe de formule OR, NHR, $NR_2$ ou NH-NH-R', où
- R représente un groupe méthyle, éthyle, n-propyle, isopropyle, n-butyle, s-butyle, isobutyle, t-butyle, trifluoro-2,2,2 éthyle, cyclohexyle, adamantyle, phényle, benzyle, fluorénylméthyle ou un groupe phénétyle;
- R' représente un atome d'hydrogène, l'un quelconque des groupes tels qu'ils sont cités dans cette revendication spécifiquement pour R, ou un groupe formyle, acétyle, trifluoroacétyle, propionyle, butyryle, adamantylcarbonyle, benzoyle, phénylacétyle, cinnamyle, benzyloxycarbonyle, nitro-4 benzyloxycarbonyle, méthoxy-4 benzyloxycarbonyle, dichloro-2,4 benzyloxycarbonyle, bromo-2 benzyloxycarbonyle, fluorényl-9 méthoxycarbonyle et diméthoxy-3,5 α,α'-diméthylbenzyloxycarbonyle, t-butoxycarbonyle, méthyl-1 cyclobutoxycarbonyle, adamantyloxycarbonyle, méthylsulfonyléthoxycarbonyle ou isobornyloxycarbonyle;

ou l'un de ses sels d'addition avec les acides ou les bases, ledit sel d'addition avec les acides provenant de l'acide sulfurique, l'acide phosphorique, l'acide chlorhydrique, l'acide bromhydrique, l'acide iodhydrique, l'acide nitrique, l'acide sulfamique, l'acide citrique, l'acide lactique, l'acide pyruvique, l'acide oxalique, l'acide maléique, l'acide succinique, l'acide tartrique, l'acide cinnamique, l'acide acétique, l'acide trifluoroacétique, l'acide benzoïque, l'acide salicylique, l'acide gluconique, l'acide ascorbique, et ledit sel d'addition avec les bases provenant de l'hydroxyde de sodium, l'hyroxyde de potassium, la diéthylamine, la triéthylamine ou la dicyclohexylamine.

3. Procédé selon la revendication 2, *caractérisé* par le fait que X représente un atome d'hydrogène.

4. Procédé selon la revendication 3, *caractérisé* par le fait que A représente un reste Met.

5. Procédé selon la revendication 1, *caractérisé* par le fait que le peptide possède la formule suivante:

H-Val-Pro-Pro-Leu-Gly-Trp-Met-OMe

ou représente un sel de ce dernier, qui est acceptable du point de vue pharmaceutique ou vétérinaire.

6. Procédé selon la revendication 1, *caractérisé* par le fait que le peptide possède la formule suivante:

H-Val-Pro-Pro-Leu-Gly-Trp-Met-OH

ou représente un sel de ce dernier, qui est acceptable du point de vue pharmaceutique ou vétérinaire.

7. Procédé selon la revendication 1, *caractérisé* par le fait que les peptides sont représentés par les formules suivantes:

H-Val-Pro-Pro-Leu-Gly-Trp-Met-$NH_2$
H-Val-Pro-Pro-Leu-Gly-Trp-Nle-OH
H-Val-Pro-Pro-Leu-Gly-Trp-Nle-OMe
H-Val-Pro-Pro-Leu-Gly-Trp-Nle-$NH_2$
H-Val-Pro-Pro-Leu-Gly-Trp-Leu-OH
H-Val-Pro-Pro-Leu-Gly-Trp-Leu-OMe
H-Val-Pro-Pro-Leu-Gly-Trp-Leu-$NH_2$
H-Val-Pro-Pro-Leu-Gly-Trp-Ile-OH
H-Val-Pro-Pro-Leu-Gly-Trp-Ile-OMe
H-Val-Pro-Pro-Leu-Gly-Trp-Ile-$NH_2$
H-Val-Pro-Pro-Leu-Gly-Trp-Phe-OH
H-Val-Pro-Pro-Leu-Gly-Trp-Phe-OMe
H-Val-Pro-Pro-Leu-Gly-Trp-Phe-$NH_2$

ou représentent leurs sels acceptables du point de vue pharmaceutique ou vétérinaire.

8. Procédé pour favoriser le taux de croissance chez les animaux, *caractérisé* par le fait qu'on administre quotidiennement, dans les rations alimentaires ou par implantation sous-cutanée, une quantité efficace d'un peptide tel que défini à la revendication 1, ou de l'un de ses sels acceptables du point de vue vétérinaire.

9. Procédé de fabrication d'un aliment pour animaux, *caractérisé* par le fait qu'on incorpore à l'aliment un peptide tel que défini à la revendication 1, ou l'un de ses sels acceptables du point de vue vétérinaire, avec un diluant ou support acceptable du point de vue vétérinaire.

10. Procédé de fabrication d'un préparation vétérinaire, *caractérisé* par le fait qu'on mélange un peptide tel que défini à la revendication 1, ou l'un de ses sels acceptables du point de vue vétérinaire, avec un diluant ou support acceptable du point de vue vétérinaire.